(19) **Europäisches Patentamt**

**European Patent Office**

**Office européen des brevets**

(11) **EP 1 426 037 B1**

(12) **FASCICULE DE BREVET EUROPEEN**

(45) Date de publication et mention
de la délivrance du brevet:
**08.03.2006 Bulletin 2006/10**

(51) Int Cl.:
*A61K 8/11* (2006.01)          *A61K 8/22* (2006.01)
*A61K 8/891* (2006.01)         *A61Q 5/06* (2006.01)
*A61Q 5/10* (2006.01)          *A61Q 17/04* (2006.01)

(21) Numéro de dépôt: **03292931.7**

(22) Date de dépôt: **26.11.2003**

(54) **Procédé de traitement cosmétique des matières kératiniques impliquant la libération contrôlée de principes actifs aromatiques à partir de particules de polymère aromatique.**

Verfahren zur kosmetischen Behandlung von keratinischen Materialen, wobei aromatische Wirkstoffe aus aromatischen Polymerpartikeln kontrolliert freigesetzt werden

Process for the cosmetic treatment of keratinic materials, involving the controlled release of aromatic active agents from aromatic polymer particles

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **04.12.2002 FR 0215298**

(43) Date de publication de la demande:
**09.06.2004 Bulletin 2004/24**

(73) Titulaire: **L'OREAL**
**75008 Paris (FR)**

(72) Inventeurs:
• **Toumi, Leila**
**95110 Sannois (FR)**
• **Bernard, Anne-Laure**
**299949 Singapour (SG)**
• **Samain, Henri**
**91570 Bievres (FR)**
• **Nicolas-Morgantini, Luc**
**60810 Rully (FR)**

(74) Mandataire: **Casalonga, Axel**
**BUREAU D.A. CASALONGA - JOSSE**
**Paul-Heyse-Strasse 33**
**80336 München (DE)**

(56) Documents cités:
**US-A- 5 298 569          US-A- 6 063 398**
**US-A- 6 083 901          US-A1- 2002 102 733**

**Description**

La présente invention concerne un procédé de traitement cosmétique des matières kératiniques et notamment des matières kératiniques humaines telles que les cheveux, les phanères et la peau, comprenant la libération contrôlée d'un principe actif cosmétique aromatique à partir de particules de polymère synthétique à groupes aryle ayant une température de transition vitreuse ($T_g$) supérieure ou égale à 45 °C, ainsi qu'un kit permettant de mettre en oeuvre un tel procédé.

L'utilisation d'actifs cosmétiques sensibles au milieu de formulation est un problème qui n'a pas encore trouvé de solution satisfaisante. Une approche consiste généralement à ajouter ledit principe actif au moment de l'emploi dans le milieu de formulation. Cette solution peut toutefois présenter des inconvénients considérabies car elle peut nécessiter des étapes de dissolution ou d'homogénéisation qui sont difficiles à mettre en oeuvre dans le cadre d'une utilisation domestique.

Par ailleurs, cette solution n'est pas satisfaisante lorsque la dégradation du principe actif par un des composants du milieu de formulation est si rapide qu'une importante fraction du principe actif est détruite avant même le procédé de traitement. Un exemple concret d'une telle situation est la coloration d'oxydation des cheveux utilisant à la fois des précurseurs de colorants d'oxydation (bases et coupleurs) et des colorants directs. Un procédé de coloration d'oxydation des cheveux implique classiquement le mélange extemporané d'une solution colorante contenant des précurseurs de colorants d'oxydation, incolores ou faiblement colorés, avec un agent oxydant qui déclenche une condensation oxydative des précurseurs aboutissant à la formation de composés colorés de masse élevée à la surface des ou dans les fibres kératiniques.

Très fréquemment, et en particulier pour l'obtention de nuances à reflets chauds (cuivrés, violines ou acajou), on ajoute aux colorants d'oxydation des colorants "directs" qui ne participent pas à la réaction d'oxydation mais interagissent directement avec les cheveux.

Les précurseurs de colorants d'oxydation sont conservés dans un compartiment séparé en présence d'un agent réducteur qui a pour fonction de stabiliser la composition en empêchant l'oxydation prématurée des précurseurs. Les agents réducteurs les plus utilisés tels que le métabisulfite de sodium et les thiols présentent toutefois l'inconvénient de dégrader un certain nombre de colorants directs ce qui interdit la conservation des deux types de colorants (colorants d'oxydation et colorants directs) dans un même compartiment.

Le document brevet US-A-6 083 901 (LIAO WEN P ET AL) décrit une composition comprenant de l'alcool phénethylique libre et un polymère synthétique à groupes aryle (polymère 2, colonne 29, lignes 23-36) et comportant des liaisons covalentes susceptibles d'être coupées par un réactif tel qu'une solution de KOH. La température de transition vitreuse du polymère n'est pas indiquée.

La demanderesse a résolu le problème de l'instabilité des colorants directs en présence de réducteurs, et plus généralement le problème que pose l'utilisation de principes actifs cosmétiques sensibles au milieu de formulation, grâce à un procédé de traitement cosmétique qui implique l'utilisation de principes actifs enfermés dans une matrice protectrice particulière, puis la libération progressive dudit principe actif par dégradation chimique de ladite matrice protectrice au moyen d'un réactif approprié.

L'incorporation de principes actifs hydrophiles, et en particulier hydrosolubles, dans des particules polymériques solides est souvent problématique car les principes actifs sont généralement incompatibles avec la matrice polymérique et l'on observe un "suintement", c'est-à-dire une lente migration des molécules de principe actif vers le milieu extérieur.

L'encapsulation des principes actifs de faible masse dans des microcapsules ou nanocapsules classiques - c'est-à-dire dans des particules constituées d'une phase liquide hydrophile entourée d'une enveloppe polymérique - obtenues soit par polymérisation interfaciale, soit par émulsion multiple eau/huile/eau suivie d'une évaporation du solvant, soit par coacervation, s'avère également dans la plupart des cas insatisfaisante car les capsules préparées relarguent généralement les principes actifs plus ou moins vite dans la phase aqueuse extérieure. Un tel relargage est également observé pour d'autres types de structures organisées telles que des vésicules ou des émulsions multiples.

La demanderesse a résolu ces problèmes pour un groupe particulier de principes actifs de faible masse; à savoir pour des principes actifs comportant un ou plusieurs noyaux aromatiques, en incorporant ces principes actifs dans des particules comprenant un polymère qui porte un certain nombre de groupes aryle et qui, à température ambiante, est à l'état vitreux. Ce polymère, pour pouvoir être dégradé et libérer le principe actif cosmétique lors du procédé de traitement cosmétique, doit comporter des liaisons covalentes susceptibles d'être coupées par un réactif approprié.

La présente invention a par conséquent pour objet un procédé de traitement cosmétique des matières kératiniques, en particulier des matières kératiniques humaines telles que les cheveux, les phanères et la peau, comprenant l'application simultanée ou consécutive de deux compositions (a) et (b),

• la composition (a) contenant des particules comprenant

(i) au moins un principe actif cosmétique, de préférence hydrosoluble, comportant un ou plusieurs groupes aromatiques, carbocycliques ou hétérocycliques, monocycliques ou polycycliques condensés, ayant un poids moléculaire inférieur ou égal à 1000, et

(ii) au moins un polymère synthétique à groupes aryle ayant une température de transition vitreuse ($T_g$) supérieure ou égale à 45 °C et comportant des liaisons covalentes susceptibles d'être coupées par un réactif R présent dans la composition (b), et

- la composition (b) comprenant au moins un réactif R capable de couper certaines liaisons covalentes du polymère arylé formant les particules de la composition (a),

la coupure chimique des liaisons du polymère arylé formant les particules présentes dans la composition (a) par le réactif R présent dans la composition (b) aboutissant à une libération du principe actif cosmétique aromatique.

Les particules de l'invention sont soit des particules composites, soit des capsules également appelées particules à structure noyau-enveloppe. Dans ce dernier cas, la paroi ou enveloppe des capsules est constituée par le ou les polymères à groupes aryle et le noyau contient le ou les actifs cosmétiques sous forme solide. De préférence les particules de l'invention sont des particules composites, c'est-à-dire des particules dans lesquelles le ou les actifs cosmétiques sont dispersés ou dissous au sein de la matrice polymérique.

L'invention a encore pour objet un kit pour le traitement cosmétique des matières kératiniques, en particulier des matières kératiniques humaines telles que les cheveux, les phanères et la peau, comportant au moins deux compartiments (A) et (B) qui contiennent respectivement les compositions (a) et (b) telles que définies ci-dessus.

Comme expliqué en introduction, le procédé objet de la présente invention et le kit permettant de le mettre en oeuvre présentent un intérêt particulier lorsque la composition (a) contient en outre un agent chimique capable de dégrader ledit principe actif cosmétique aromatique. La présence de cet agent chimique dans la composition (a) n'est toutefois pas essentielle pour l'invention car l'agent capable de dégrader le principe actif cosmétique peut tout aussi bien être un agent physico-chimique extérieur tel que la température ambiante ou le rayonnement solaire. Ainsi, le procédé de l'invention pourrait servir à prolonger l'action d'une composition solaire en libérant progressivement des filtres solaires photosensibles qui se trouvent photostabilisés par encapsulation dans les particules de polymères arylés décrits ci-après.

Le terme "polymères synthétiques à groupes aryle" utilisé pour désigner les polymères servant à la fabrication des particules utilisées dans la présente invention englobe des polymères comportant un certain nombre de groupes aryle et/ou arylène, de préférence phényle et/ou phénylène, les groupes arylène pouvant former la chaîne principale du polymère ou bien faire partie des chaînes latérales. Il peut s'agir d'homopolymères ou de copolymères séquencés, statistiques ou alternés, obtenus par polyaddition ou par polycondensation. Ce terme englobe également les polymères de greffage ayant une structure ramifiée ou réticulée.

Les polymères arylés formant la matrice ou l'enveloppe des particules utilisées dans la présente invention, doivent, bien entendu, être insolubles dans l'eau et dans la plupart des solvants cosmétiquement acceptables. Les polymères arylés utilisés sont par conséquent de préférence non-ioniques, c'est-à-dire exempts de charges susceptibles de les rendre solubles dans l'eau ou dans d'autres solvants polaires.

Comme indiquée ci-dessus, la température de transition vitreuse du polymère formant la matrice ou l'enveloppe des particules utilisées dans le procédé de la présente invention doit au moins être égale à 45 °C. Elle est de préférence supérieure ou égale à 50 °C et idéalement supérieure ou égale à 60 °C. Ce paramètre est essentiel pour l'invention. En effet, il s'est avéré que le caractère figé, vitreux du polymère à température ambiante était une condition indispensable pour empêcher la migration des molécules de principe actif encapsulées vers la surface des particules ou bien la pénétration de l'agent chimique à l'intérieur des particules. Or, les particules utilisées dans la présente invention sont susceptibles d'être exposées à des températures relativement élevées qui règnent par exemple l'été à la plage. Lorsque la température des particules s'approche alors de la température de transition vitreuse du polymère, celui-ci se plastifie et il y a un risque de fuite du principe actif hydrophile vers l'extérieur des particules.

Comme expliqué ci-dessus, le polymère arylé utilisé pour la protection des principes actifs aromatiques ne doit pas seulement permettre une encapsulation satisfaisante du principe actif, mais doit également se dégrader assez rapidement en présence d'un réactif approprié de manière à permettre la libération du principe actif au cours du procédé de traitement cosmétique.

Dans un mode de réalisation préféré de l'invention, les liaisons covalentes du polymère arylé, susceptibles d'être coupées par le réactif R sont des liaisons Si-C$_{aromatique}$, telles que Si-phényle, et/ou des liaisons disulfure (liaisons S-S).

La demanderesse a observé en effet, que ces deux types de liaisons, même lorsqu'elles font partie de la chaîne macromoléculaire d'un polymère solide, insoluble dans le milieu réactionnel, peuvent être coupés rapidement par exemple en présence de peroxyde d'hydrogène.

Dans le cas d'un procédé de coloration d'oxydation des cheveux - mode de réalisation préféré du procédé de l'invention - ce dernier composé joue alors à la fois le rôle de réactif R (dégradation du polymère arylé) et celui d'agent oxydant permettant la condensation oxydative des précurseurs de colorants d'oxydation.

Un autre réactif R, capable de couper des liaisons Si-C$_{aromatique}$, englobe les ions fluorure, par exemple sous forme de sels de fluor tels que le fluorure de sodium, de potassium ou de tétrabutylammonium. Les sels de fluor peuvent également être utilisés en combinaison avec l'eau oxygénée (H$_2$O$_2$) et augmentent alors la vitesse de coupure des liaisons Si-C$_{aromatique}$ et accélèrent la libération du principe actif hydrosoluble.

Un groupe de polymères arylés préférés selon l'invention est formé par les polyorganosiloxanes comportant des groupes aryle, de préférence phényle, portés directement par les atomes de silicium du squelette siloxane. Les polyorganosiloxanes phénylés peuvent avoir une structure linéaire, ramifiée ou réticulée.

La teneur appropriée en groupes aryle, et en particulier phényle, des polyorganosiloxanes dépend, entre autres, de la quantité de principe actif à incorporer. En effet, la demanderesse a constaté que, pour un type de polymère donné, la quantité de principe actif qu'il est possible d'encapsuler de manière stable semble augmenter avec la teneur en groupes aryle. Par ailleurs, la teneur en groupes aryle a une influence sur la température de transition vitreuse, celle-ci étant généralement plus élevée pour des teneurs en groupes aryle plus importantes.

La demanderesse a obtenu des résultats satisfaisants en particulier pour une teneur en groupes aryle telle que le rapport du nombre de groupes aryle au nombre d'atomes de silicium est comprise entre 1/15 et 2/1. On préfère tout particulièrement des polyorganosiloxanes ayant un rapport du nombre de groupes aryle au nombre d'atomes de silicium compris dans l'intervalle allant de 1/10 à 2/1.

On peut citer à titre d'exemple de tels polyorganosiloxanes arylés le polymère DC(R) Z-6018, un phénylpropylsilsesquioxane commercialisé par la société Dow Chemical.

Une autre famille de polymères arylés utilisables pour la stabilisation et libération contrôlée de principes actifs aromatiques englobe les polycondensats obtenus à partir de monomères arylés comportant des liaisons disulfure. Ces polycondensats sont généralement des copolymères constitués d'au moins deux types de monomères différents. Les groupes aryle et les liaisons S-S peuvent alors être apportés par un même monomère ou par deux ou plus de deux monomères différents.

Des exemples de monomères comportant une liaison S-S ou à la fois un groupe aryle et une liaison S-S englobent, par exemple, le bis-4-aminophényldisulfure, l'homocystine, la cystamine, le formamidine-disulfure et le di-(2-hydroxyéthyl)disulfure.

On peut citer à titre d'exemples de tels polycondensats les polyuréthanes obtenus par polycondensation d'au moins un diisocyanate et d'au moins un composé comportant deux fonctions à hydrogène labile, choisies par exemple parmi les fonctions hydroxyle, thiol, amine primaire et amine secondaire, les monomères étant choisis de manière à ce qu'au moins un type de comonomères comporte un groupe aryle et au moins un comonomère comporte une liaison disulfure (S-S). Il ressort de ce qui précède que le terme "polyuréthanes", tels qu'il est utilisé dans la présente demande, englobe non seulement les polyuréthanes proprement dits comportant des liaisons carbamate (-NH-CO-O-), mais également les polyurées (à liaisons -NH-CO-NH- ou -NR-CO-NH-) et les polythiourées (à liaisons NH-CO-S-).

Les polycondensats arylés peuvent également être des polyesters ou polyamides obtenus par polycondensation d'au moins un diacide ou d'un dérivé activé d'un diacide, avec respectivement au moins un diol ou au moins une diamine, ces monomères étant choisis, comme précédemment, de manière à ce qu'au moins un des types de monomères comporte un groupe aryle et au moins un type de monomère comporte une liaison disulfure.

Les monomères donnant les polycondensats arylés ci-dessus, à savoir les polyuréthannes, polyesters, polyamides et copolymères de ceux-ci, sont connus dans la technique.

On peut citer à titre d'exemple de monomères non-arylés :

- les diisocyanates aliphatique tels que l'hexaméthylènediisocyanate et l'isophorone-diisocyanate,
- les diols aliphatiques tels que les alkylèneglycols ou polyalkylèneglycols,
- les diamines aliphatiques telles que les alkylènediamines,
- les diacides aliphatique tels que l'acide succinique, l'acide fumarique, l'acide adipique et l'acide sébacique, ou les anhydrides ou chlorures de ces diacides,
- les aminoalcools,
- les aminoacides.

A titre d'exemples de monomères arylés, on peut citer

- les diisocyanates aromatiques tels que le toluènediisocyanate et le diphénylméthanediisocyanate,
- les diamines aromatiques telles que les phénylènediamines, éventuellement substituées,
- les diols aromatiques tels que les bisphénols, par exemple le bisphénol A,
- les diacides aromatiques tels que l'acide téréphtalique et l'acide isophtalique.

La polycondensation des monomères divalents énumérés ci-dessus aboutit à des structures linéaires. Pour l'obtention de structures ramifiées, on peut ajouter une certaine fraction de monomères au moins trivalents, tels que le trichlorure d'acide 1,3,5-benzènetricarboxylique, le tétraisocyanate de silicium, la 2,4,6-triamino-1,3,5-triazine, le glycérol, le polyglycérol, ou le glycérol polypropoxylé à terminaisons amino.

Les particules de la présente invention peuvent avoir des structures et des tailles très variables découlant de leur procédé de préparation et liées à l'application envisagée.

Il peut s'agir par exemple de microparticules obtenues par broyage d'un matériau solide constitué d'une matrice de polymère phénylé dans laquelle sont finement dispersés ou dissous (solution solide) le ou les principe(s) actif(s) aromatique(s). Ces microparticules ont de préférence une taille moyenne comprise entre 0,05 et 500 $\mu$m.

D'autres microparticules ayant une taille comprise entre 0,05 et 500 $\mu$m peuvent être obtenues par émulsion multiple. Elles ont une structure de type noyau-enveloppe dans laquelle une enveloppe formée par le polymère arylé entoure hermétiquement un noyau liquide contenant le principe actif. La technique de l'émulsion multiple utilisée pour la formation de ce type de particules est décrite par exemple dans les demandes FR 2 766 737 et DE 197 19 297.

Dans un autre mode de réalisation, les particules sont des particules à structure noyau-enveloppe ayant une taille relativement plus importante, comprise de préférence entre 0,5 et 10 mm. Ces particules sont obtenues par coextrusion de deux phases, la phase extérieure étant formée par le polymère arylé, à l'état fondu ou sous forme de solution visqueuse ayant de préférence une viscosité supérieure à 1000 centipoises (mesurée à 20 °C), et la phase interne contenant le principe actif à l'état fondu ou sous forme de solution épaisse.

Pour protéger efficacement le principe actif dans la matrice polymérique des particules de l'invention, il est préférable que le rapport en poids du principe actif au polymère arylé ne dépasse pas une certaine valeur limite qui dépend par exemple du taux de groupes aryle du polymère. Plus celui-ci est important, plus la limite supérieure du rapport en poids principe actif/polymère arylé donnant lieu à une protection efficace est élevée. La demanderesse a constaté que l'on obtient en particulier des résultats d'encapsulation très satisfaisants pour un rapport en poids du principe actif hydrosoluble aromatique au polymère arylé compris entre 1/1 et 1/50, de préférence entre 1/3 et 1/20.

Les particules de polymère arylé contenant un principe actif aromatique hydrosoluble peuvent être préparées selon différentes techniques connues comprenant l'incorporation dudit principe actif aromatique dans une matrice d'un polymère synthétique à groupes aryle ou l'enrobage ou l'encapsulation d'un tel principe actif hydrosoluble aromatique dans une enveloppe formée d'un tel polymère à groupes aryle.

L'incorporation du principe actif dans une matrice peut se faire soit par fusion soit par dissolution du polymère et du principe actif.

Le procédé par fusion comprend les étapes suivantes consistant

- à faire fondre le polymère synthétique à groupes aryle,
- à dissoudre ou à disperser finement le principe actif aromatique dans le polymère fondu,
- à laisser refroidir le mélange ainsi obtenu jusqu'à température ambiante, et
- à broyer le matériau solide jusqu'à une taille de particules appropriée, de préférence comprise entre 0,05 et 500 $\mu$m.

Ce procédé présente l'avantage de pouvoir se faire en l'absence de solvants organiques. Il présente toutefois la difficulté de ne pouvoir être mis en oeuvre qu'avec des polymères ayant une température de fusion inférieure à la température de décomposition du principe actif.

Le procédé par dissolution permet de remédier à cet inconvénient. Ce procédé comprend les étapes suivantes consistant

- à dissoudre le polymère synthétique à groupes aryle et le principe actif aromatique dans un solvant ou mélange de solvants approprié,
- à évaporer le solvant de manière à obtenir un matériau solide, et
- à broyer le matériau solide jusqu'à une taille de particules appropriée, de préférence comprise entre 0,05 et 500 $\mu$m.

Ce procédé nécessite la sélection d'un solvant ou mélange de solvants permettant de dissoudre à la fois le polymère arylé hydrophobe et le principe actif qui est souvent hydrophile.

On peut citer à titre d'exemples de tels solvants ou mélanges de solvants les alcools en C$_{1-4}$ tels que le méthanol, l'éthanol ou l'isopropanol. On peut aussi utiliser d'autres solvants organiques tels que le DMSO et le tétrahydrofurane.

Le broyage du matériau solide vitreux obtenu après refroidissement à température ambiante ou après évaporation du solvant, peut se faire à l'aide de n'importe quel type de broyeur connu dans la technique permettant d'obtenir des particules ayant la taille souhaitée, de préférence comprise entre 0,05 et 500 $\mu$m. On peut citer à titre d'exemples les broyeurs à couteaux, les broyeurs à billes, les broyeurs à broches.

Enfin, un troisième type de procédé comprend la coextrusion du principe actif aromatique et du polymère synthétique à groupes aryle de manière à obtenir des particules constituées d'un noyau solide formé par le principe actif hydrosoluble aromatique, et d'une enveloppe solide formée par le polymère synthétique à groupes aryle.

La coextrusion du polymère arylé et du principe actif aromatique se fait selon un procédé connu décrit par exemple dans Harper J.M., 1990, *Extrusion of foods in biotechnology and food process engineering,* éditeur Marcel DEKKER Inc. chapitre 10, page 307.

Les principes actifs cosmétiques sont choisis parmi les principes actifs aromatiques de faible poids moléculaire c'est-à-dire inférieur à 1000 et de préférence inférieur à 500. Ces principes actifs aromatiques de faible masse sont de préférence hydrosolubles.

On entend par principe actif hydrosoluble selon l'invention un principe actif ayant une solubilité dans l'eau, mesurée à 25 °C, au moins égale à 0,1 g/l (obtention d'une solution macroscopiquement isotrope et transparente, colorée ou non). Cette solubilité est de préférence supérieure ou égale à 1 g/l.

Le caractère hydrophile des principes actifs aromatiques peut être apporté par un ou plusieurs substituants ionisables ou ionisés, tels que des groupes sulfonate, sulfate, carboxylate, phosphonate, phosphate, amine primaire, secondaire ou tertiaire ou un groupe ammonium

On peut citer à titre d'exemples de tels principes actifs cosmétiques

- les colorants tels que les colorants directs, les précurseurs de colorants d'oxydation (bases ou coupleurs),
- les filtres UV-A et UV-B organiques, tels que les dibenzoylméthanes, les benzimidazoles, les benzophénones et les benzotriazoles
- les flavonoïdes tels que ceux contenus dans les extraits de seigle,
- les vitamines telles que la vitamine B12,
- les agents réducteurs,
- les acides tels que l'acide salicylique.

Parmi ces principes actifs, on préfère en particulier les colorants capillaires directs et en particulier les colorants directs azoïques, les colorants directs nitrés, les colorants directs anthraquinoniques ou naphtaléniques, les colorants directs triarylméthiniques, les filtres UV organiques et les flavonoïdes. Les colorants directs peuvent être de nature non-ionique, anionique, cationique ou amphotère.

Comme expliqué en introduction, le procédé de traitement cosmétique de la présente invention est particulièrement intéressant dans le domaine de la coloration d'oxydation des cheveux utilisant à la fois des précurseurs de colorants d'oxydation et des colorants directs sensibles à l'agent réducteur (par exemple le métabisulfite de sodium) destiné à prévenir l'oxydation prématurée des précurseurs de colorants d'oxydation.

Par conséquent, dans un mode de réalisation particulièrement préféré du procédé de l'invention, on mélange, immédiatement avant emploi,

- une composition colorante comprenant, dans un milieu cosmétiquement acceptable, un ou plusieurs précurseurs de colorants d'oxydation, un agent réducteur, et un colorant aromatique, de préférence hydrosoluble, susceptible d'être dégradé par ledit agent réducteur, ce colorant étant enfermé dans des particules d'un polymère synthétique à groupes aryle ayant une température de transition vitreuse ($T_g$) supérieure ou égale à 45 °C et comportant des liaisons S-S et/ou des liaisons Si-C$_{aromatique}$, et
- une composition oxydante contenant du peroxyde d'hydrogène,

on applique le mélange ainsi obtenu sur les fibres kératiniques, on laisse reposer pendant un temps suffisant pour obtenir la coloration souhaitée, et l'on rince, lave et sèche les fibres kératiniques.

L'invention a également pour objet des kits multi-compartiments permettant de mettre en oeuvre le procédé de traitement cosmétique décrit ci-dessus. Un tel kit multi-compartiments selon l'invention comporte au moins deux compartiments (A) et (B), le compartiment (A) contenant une composition (a) renfermant des particules comprenant

(i) au moins un principe actif cosmétique, de préférence hydrosoluble, comportant un ou plusieurs groupes aromatiques, carbocycliques ou hétérocycliques, monocycliques ou polycycliques condensés, ayant un poids moléculaire inférieur ou égal à 1000, et

(ii) au moins un polymère synthétique à groupes aryle ayant une température de transition vitreuse ($T_g$) supérieure ou égale à 45 °C et comportant des liaisons covalentes susceptibles d'être coupées par un réactif R présent dans la composition (b),

et le compartiment (B) contenant une composition (b) qui contient au moins un réactif R capable de couper certaines liaisons covalentes du polymère arylé formant les particules de la composition (a).

Dans un mode de réalisation préféré de l'invention, le kit multi-compartiments est un kit de coloration d'oxydation des cheveux dans lequel la composition (a) contient, dans un milieu cosmétiquement acceptable, un ou plusieurs précurseurs de colorants d'oxydation, un réducteur et un colorant aromatique, de préférence hydrosoluble, susceptible d'être dégradé par le réducteur, ce colorant étant enfermé dans des particules d'un polymère synthétique à groupes aryle ayant une température de transition vitreuse (T$_g$) supérieure ou égale à 45 °C et comportant des liaisons S-S et/ou des liaisons Si-phényle, et la composition (b) contient du peroxyde d'hydrogène.

Les exemples ci-après illustrent la préparation de particules de polymère arylé contenant un principe actif aromatique hydrosoluble (exemple 1) et un procédé de traitement cosmétique avec libération contrôlée du principe actif par dégradation chimique des particules contenant celui-ci (exemple 2).

**Exemple 1**

Encapsulation d'un principe actif cosmétique hydrosoluble

On disperse 1 partie en poids d'une poudre de colorant azoïque hydrosoluble ayant une masse moléculaire inférieure à 300 g/mole (MIP RED 2985-2, Ciba) dans 10 parties en poids d'un phénylpropylsilsesquioxane ayant une masse de 1500 - 2500 g/mole (DC® Z-6018, Dow Corning) à l'état fondu (130 °C) et l'on mélange de manière à obtenir une suspension homogène. On laisse refroidir le mélange juscju'à température ambiante, puis on le broie à l'aide d'un mortier jusqu'à une taille moyenne de particules d'environ 500 μm. La poudre ainsi préparée est ensuite lavée 3 fois, pendant 24 heures sous agitation, dans une solution aqueuse, puis on la disperse dans de l'eau à l'aide d'un dispersant siliconé (dimethicone copolyol isostearate).

Ce lavage a pour objectif d'éliminer les molécules de colorant qui se retrouvent à la surface des particules broyées. Un dosage de la quantité de colorant contenue dans les particules ainsi lavées révèle que 8 % du colorant ont été éliminés par lavage, c'est-à-dire le taux d'encapsulation est de 92 %.

Efficacité de la protection du colorant encapsulé

La poudre est ensuite dispersée, à l'aide du dispersant siliconé ci-dessus, dans une solution ammoniacale à pH 10,5 contenant 0,53 % en poids de métabisulfite de sodium, et dans une solution ammoniacale à pH 10,5 exempt d'agent réducteur.

Après 2 mois de conservation de ces deux suspensions à une température de 45 °C, le dosage de la quantité de colorant ayant fui dans le milieu aqueux ammoniacal exempt de métabisulfite indique un taux d'encapsulation de 90 %, au lieu des 92 % mesurés initialement, c'est-à-dire une perte d'environ 2 % de la quantité de colorant initialement encapsulée.

Le dosage de la quantité de colorant restant dans les particules conservées pendant 2 mois à 45 °C dans la solution de métabisulfite de sodium, par spectrométrie UV-visible d'une solution de la poudre dans un mélange chloroforme/méthanol (9/1), révèle une perte de colorant d'environ 5 % (87 % de colorant restant au lieu des 92 % initialement encapsulés).

Le fait que la perte de colorant en présence d'agent réducteur (5 %) est légèrement supérieure à celle observée en l'absence d'agent réducteur (2 %) montre qu'une faible fraction de colorant encapsulé a été détruite par l'agent réducteur. Ce résultat peut être attribué à la très grande surface spécifique de la poudre finement dispersée qui favorise les échanges entre la surface des particules et le milieu environnant.

A titre de comparaison, si le colorant est directement introduit dans la solution ammoniacale à pH 10,5 contenant le bisulfite de sodium (donc sans protection par les particules), on constate qu'il est totalement détruit après 2 mois à 45 °C.

Cet exemple montre que le colorant est encapsulé avec un bon taux d'encapsulation et se trouve protégé efficacement contre la dégradation chimique par le métabisulfite de sodium.

**Exemple 2**

Kit de coloration des cheveux et procédé de coloration

Compartiment A
Colorant direct (MIP RED 2985-2, Ciba)
encapsulé conformément à l'exemple 1 — 0,1 g m.a.
NH$_4$OH — 2 g
Métabisulfite de sodium — 0,53 g m.a.
Eau — q.s.p. 100 g

Suite de tableau

Compartiment B

Eau oxygénée                                              à 20 volumes

On mélange 1 partie en volume de la composition du compartiment A avec 1 partie en volume de la composition du compartiment B. Le mélange obtenu a un pH de 9,53. Après 10 minutes, on applique le mélange sur une mèche de cheveux permanentés à 90 % de blancs avec un rapport en poids composition colorante/mèche d'environ 10.

Après un temps de pose de 30 minutes à température ambiante, on rince la mèche avec de l'eau et on la sèche.

Le tableau 1 ci-dessous rassemble les paramètres colorimétriques du système CIE L*a*b* et du système TSL (Teinte, Saturation, Luminosité) (en anglais HSL), mesurés à l'aide d'un colorimètre MINOLTA CM3600d (illuminant D65-10° CSE).

L'écart de couleur ΔE entre la mèche d'origine et la mèche teinte est calculé selon la formule

$$\Delta E = \sqrt{(L*_{final} - L*_{initial})^2 + (a*_{final} - a*_{initial})^2 + (b*_{final} - b*_{initial})^2}$$

Les valeurs obtenues avec la composition de coloration selon l'invention, préparée de la manière décrite ci-dessus, sont comparées à celles obtenues, dans les mêmes conditions, avec un mélange de la composition du compartiment A ci-dessus et d'une solution d'eau acidifiée avec de l'acide phosphorique, exempte de réactif R ($H_2O_2$) capable de dégrader le polymère formant les particules. Le pH du mélange comparatif appliqué sur les cheveux est de 9,85.

|  | L* | a* | b* | h (teinte) | ΔE |
|---|---|---|---|---|---|
| avant coloration | 59,97 | 1,82 | 11,12 | 80,70 | |
| coloration selon l'invention | 57,43 | 5,78 | 8,52 | 55,86 | 5,37 |
| coloration comparative | 59,56 | 1,83 | 9,83 | 79,45 | 1,35 |

Cet exemple montre clairement que seules les particules mises en contact avec l'eau oxygénée se dégradent rapidement et libèrent le colorant direct encapsulé.

Dans le but de mettre en évidence la libération du colorant direct encapsulé dans un polymère arylé, cet exemple a été réalisé avec un colorant direct en l'absence de précurseurs de colorants d'oxydation.

Il va de soi que la libération du colorant direct par les particules de polymère arylé au contact de l'eau oxygénée, pourra être avantageusement mise à profit dans un procédé de teinture d'oxydation utilisant des précurseurs de colorants d'oxydation connus (bases et coupleurs) qui seraient alors présents dans le compartiment A du kit utilisé ci-dessus.

**Revendications**

**1.** Procédé de traitement cosmétique des matières kératiniques, en particulier des matières kératiniques humaines telles que les cheveux, les phanères et la peau, comprenant l'application simultanée ou consécutive de deux compositions (a) et (b),

    • la composition (a) contenant des particules comprenant

        (i) au moins un principe actif cosmétique comportant un ou plusieurs groupes aromatiques, carbocycliques ou hétérocycliques, monocycliques ou polycycliques condensés, ayant un poids moléculaire inférieur ou égal à 1000, et

        (ii) au moins un polymère synthétique à groupes aryle ayant une température de transition vitreuse ($T_g$) supérieure ou égale à 45 °C et comportant des liaisons covalentes susceptibles d'être coupées par un réactif R présent dans la composition (b), et

    • la composition (b) comprenant au moins un réactif R capable de couper certaines liaisons covalentes du polymère arylé formant les particules de la composition (a),

la coupure chimique des liaisons du polymère arylé formant les particules présentes dans la composition (a) par le réactif R présent dans la composition (b) aboutissant à une libération du principe actif cosmétique aromatique.

2. Procédé selon la revendication 1, **caractérisé par le fait que** le ou les principe(s) actif(s) cosmétique(s) (i) sont hydrosolubles.

3. Procédé selon la revendication 1 ou 2, **caractérisé par le fait que** les particules sont des particules composites dans lesquelles le ou les principes actifs cosmétiques (i) sont dispersés ou dissous au sein de la matrice polymérique.

4. Procédé selon l'une quelconque des revendications précédentes, **caractérisé par le fait que** la composition (a) contient en outre un agent chimique capable de dégrader ledit principe actif cosmétique.

5. Procédé selon l'une quelconque des revendications précédentes, **caractérisé par le fait que** le polymère synthétique à groupes aryle est un polymère non-ionique.

6. Procédé selon l'une quelconque des revendications précédentes, **caractérisé par le fait que** la température de transition vitreuse du polymère synthétique à groupes aryle est supérieure à 50 °C, de préférence supérieure à 60 °C.

7. Procédé selon l'une quelconque des revendications précédentes, **caractérisé par le fait que** les liaisons covalentes du polymère arylé, susceptibles d'être coupées par le réactif R, sont des liaisons Si-C$_{aromatique}$ et/ou des liaisons disulfure (S-S).

8. Procédé selon la revendication 7, **caractérisé par le fait que** polymère à groupes aryle est un polyorganosiloxane comportant des groupes aryle liés directement aux atomes de silicium du squelette siloxane.

9. Procédé selon la revendication 8, **caractérisé par le fait que** le rapport du nombre de groupes aryle au nombre d'atomes de silicium du polymère est compris entre 1/15 et 2/1.

10. Procédé selon la revendication 7, **caractérisé par le fait que** le polymère synthétique à groupes aryle est un polyuréthanne obtenu par polycondensation d'au moins un diisocyanate et d'au moins un composé comportant deux fonctions à hydrogène labile, ces monomères étant choisis de manière à ce qu'au moins un type de comonomères comporte un groupe aryle et qu'au moins un type de comonomères comporte une liaison disulfure (S-S).

11. Procédé selon la revendication 7, **caractérisé par le fait que** le polymère synthétique à groupes aryle est un polyester ou polyamide obtenu par polycondensation d'au moins un diacide ou d'un dérivé activé d'un diacide, et respectivement d'au moins un diol ou d'au moins une diamine, ces comonomères étant choisis de manière à ce qu'au moins un type de comononomères comporte un groupe aryle et qu'au moins un type de comonomères comporte une liaison disulfure (S-S).

12. Procédé selon l'une quelconque des revendications précédentes, **caractérisé par le fait que** le réactif R est le peroxyde d'hydrogène (H$_2$O$_2$).

13. Procédé selon l'une quelconque des revendications précédentes, **caractérisé par le fait que** le principe actif cosmétique aromatique a un poids moléculaire inférieur ou égal à 500.

14. Procédé selon l'une quelconque des revendications précédentes, **caractérisé par le fait que** le principe actif cosmétique aromatique porte au moins un groupe ionisé ou ionisable.

15. Procédé selon l'une quelconque des revendications précédentes, **caractérisé par le fait que** le rapport en poids du principe actif cosmétique au polymère arylé est compris entre 1/1 et 1/50, de préférence entre 1/3 et 1/20.

16. Procédé selon l'une quelconque des revendications précédentes, **caractérisé par le fait que** le principe actif cosmétique est un colorant capillaire direct, un précurseur de colorant d'oxydation, un filtre UV organique ou un flavonoïde.

17. Procédé selon l'une quelconque des revendications précédentes, **caractérisé par le fait que** les particules présentes dans la composition (a) sont des microparticules ayant une taille moyenne comprise entre 0,05 et 500 μm ou des particules ayant une taille moyenne comprise entre 0,5 et 10 mm.

18. Procédé selon la revendication 17, **caractérisé par le fait que** les particules ou les microparticules ont une structure noyau-enveloppe dans laquelle le noyau est formé par ou contient ledit principe actif cosmétique aromatique et l'enveloppe comprend ou est formée par ledit polymère synthétique à groupes aryle.

19. Procédé selon l'une quelconque des revendications précédentes, **caractérisé par le fait que** l'on mélange, immédiatement avant emploi,

• une composition colorante comprenant, dans un milieu cosmétiquement acceptable, un ou plusieurs précurseurs de colorants d'oxydation, un agent réducteur et un colorant aromatique susceptible d'être dégradé par l'agent réducteur, ce colorant étant enfermé dans des particules d'un polymère synthétique à groupes aryle ayant une température de transition vitreuse ($T_g$) supérieure ou égale à 45 °C et comportant des liaisons S-S et/ou des liaisons Si-C$_{aromatique}$, et
• une composition oxydante contenant du peroxyde d'hydrogène,

que l'on applique le mélange ainsi obtenu sur les fibres kératiniques, que l'on laisse reposer pendant un temps suffisant pour obtenir la coloration souhaitée, et que l'on rince, lave et sèche les fibres kératiniques.

20. Procédé selon la revendication 19, **caractérisé par le fait que** l'agent réducteur est le métabisulfite de sodium ou un thiol.

21. Kit pour le traitement cosmétique des matières kératiniques, en particulier des matières kératiniques humaines telles que les cheveux, les phanères et la peau, comportant au moins deux compartiments (A) et (B), qui contiennent respectivement les compositions (a) et (b) telles que définies dans l'une quelconque des revendications précédentes.

22. Kit selon la revendication 21, **caractérisé par le fait que** la composition (a) contient, dans un milieu cosmétiquement acceptable, un ou plusieurs précurseurs de colorants d'oxydation, un réducteur et un colorant aromatique, de préférence hydrosoluble, susceptible d'être dégradé par le réducteur, ce colorant étant enfermé dans des particules d'un polymère synthétique à groupes aryle ayant une température de transition vitreuse ($T_g$) supérieure ou égale à 45 °C et comportant des liaisons S-S et/ou des liaisons Si-phényle, et que la composition (b) contient du peroxyde d'hydrogène.

**Patentansprüche**

1. Verfahren zur kosmetischen Behandlung von Keratinsubstanzen, insbesondere menschlichen Keratinsubstanzen, wie Haaren, Hautanhangsgebilden und der Haut, das das gleichzeitige oder aufeinander folgende Aufbringen von zwei Zusammensetzungen (a) und (b) umfasst,

• wobei die Zusammensetzung (a) Partikel enthält, die umfassen:

(i) mindestens einen kosmetischen Wirkstoff, der eine oder mehrere aromatische, carbocyclische oder heterocyclische, kondensierte polycyclische oder monocyclische Gruppen mit einer Molmasse von 1.000 oder darunter aufweist, und
(ii) mindestens ein synthetisches Polymer mit Arylgruppen und einer Glasübergangstemperatur ($T_g$) von 45 °C oder darüber, das kovalente Bindungen aufweist, die mit einem in der Zusammensetzung (b) enthaltenen Reaktanten R gespalten werden können, und

• die Zusammensetzung (b) mindestens einen Reaktanten R enthält, der befähigt ist, bestimmte kovalente Bindungen des arylierten Polymers zu spalten, das die Partikel der Zusammensetzung (a) bildet,

wobei das chemische Spalten der Bindungen des arylierten Polymers, das die in der Zusammensetzung (a) vorliegenden Partikel bildet, durch den in der Zusammensetzung (b) enthaltenen Reaktanten R dazu führt, dass der aromatische kosmetische Wirkstoff freigesetzt wird.

2. Verfahren nach Anspruch 1, **dadurch gekennzeichnet, dass** der oder die kosmetische(n) Wirkstoff(e) wasserlöslich sind.

3. Verfahren nach Anspruch 1 oder 2, **dadurch gekennzeichnet, dass** die Partikel Verbundpartikel sind, bei denen der oder die kosmetischen Wirkstoffe (i) in der Polymermatrix dispergiert oder gelöst sind.

4. Verfahren nach einem der vorhergehenden Ansprüche, **dadurch gekennzeichnet, dass** die Zusammensetzung (a) ferner einen chemischen Stoff enthält, der befähigt ist, den kosmetischen Wirkstoff zu zersetzen.

5. Verfahren nach einem der vorhergehenden Ansprüche, **dadurch gekennzeichnet, dass** das synthetische Polymer mit Arylgruppen ein nichtionisches Polymer ist.

6. Verfahren nach einem der vorhergehenden Ansprüche, **dadurch gekennzeichnet, dass** die Glasübergangstemperatur des synthetischen Polymers mit Arylgruppen über 50 °C und vorzugsweise über 60 °C liegt.

7. Verfahren nach einem der vorhergehenden Ansprüche, **dadurch gekennzeichnet, dass** die kovalenten Bindungen des arylierten Polymers, die von dem Reaktanten R gespalten werden können, Bindungen $Si-C_{aromatisch}$ und/oder Disulfidbindungen (S-S) sind.

8. Verfahren nach Anspruch 7, **dadurch gekennzeichnet, dass** das Polymer mit Arylgruppen ein Polyorganosiloxan ist, das arylierte Gruppen aufweist, die direkt an Siliciumatome des Siloxangerüsts gebunden sind.

9. Verfahren nach Anspruch 8, **dadurch gekennzeichnet, dass** das Verhältnis der Anzahl der Arylgruppen und der Anzahl der Siliciumatome des Polymers im Bereich von 1/15 bis 2/1 liegt.

10. Verfahren nach Anspruch 7, **dadurch gekennzeichnet, dass** das synthetische Polymer mit Arylgruppen ein Polyurethan ist, das durch Polykondensation mindestens eines Diisocyanats und mindestens einer Verbindung erhalten wird, die zwei Funktionen mit labilem Wasserstoff aufweist, wobei die Monomere so ausgewählt sind, dass mindestens ein Typ von Comonomeren eine Arylgruppe aufweist und mindestens ein Typ von Comonomeren eine Disulfidbindung (S-S) besitzt.

11. Verfahren nach Anspruch 7, **dadurch gekennzeichnet, dass** das synthetische Polymer mit Arylgruppen ein Polyester oder Polyamid ist, die durch Polykondensation mindestens einer Disäure oder eines aktivierten Disäurederivats und mindestens eines Diols bzw. mindestens eines Diamins hergestellt werden, wobei die Comonomere so ausgewählt sind, dass mindestens ein Typ von Comonomeren eine Arylgruppe aufweist und mindestens ein Typ von Comonomeren eine Disulfidbindung (S-S) besitzt.

12. Verfahren nach einem der vorhergehenden Ansprüche, **dadurch gekennzeichnet, dass** es sich bei dem Reaktanten R um Wasserstoffperoxid ($H_2O_2$) handelt.

13. Verfahren nach einem der vorhergehenden Ansprüche, **dadurch gekennzeichnet, dass** der aromatische kosmetische Wirkstoff ein Molekulargewicht von 500 oder darunter aufweist.

14. Verfahren nach einem der vorhergehenden Ansprüche, **dadurch gekennzeichnet, dass** der aromatische kosmetische Wirkstoff mindestens eine ionisierbare oder ionisierte Gruppe aufweist.

15. Verfahren nach einem der vorhergehenden Ansprüche, **dadurch gekennzeichnet, dass** das Gewichtsverhältnis des kosmetischen Wirkstoffs und des arylierten Polymers im Bereich von 1 / 1 bis 1/50 und vorzugsweise 1/3 bis 1/20 liegt.

16. Verfahren nach einem der vorhergehenden Ansprüche, **dadurch gekennzeichnet, dass** der kosmetische Wirkstoff ein Direktfarbstoff für das Haar, ein Farbstoffvorprodukt eines Oxidationsfarbstoffes, ein organisches UV-Filter oder ein Flavonoid ist.

17. Verfahren nach einem der vorhergehenden Ansprüche, **dadurch gekennzeichnet, dass** die in der Zusammensetzung (a) enthaltenen Partikel Mikropartikel mit einer mittleren Größe von 0,05 bis 500 $\mu$m oder Partikel mit einer mittleren Größe von 0,5 bis 10 mm sind.

18. Verfahren nach Anspruch 17, **dadurch gekennzeichnet, dass** die Partikel oder Mikropartikel eine Kern-Schale-Struktur aufweisen, bei der der Kern durch den kosmetischen aromatischen Wirkstoff gebildet wird oder den kosmetischen aromatischen Wirkstoff enthält und die Hülle von dem synthetischen Polymer mit Arylgruppen gebildet wird oder das synthetische Polymer mit Arylgruppen enthält.

19. Verfahren nach einem der vorhergehenden Ansprüche, **dadurch gekennzeichnet, dass** unmittelbar vor der Anwendung vermischt werden:

• eine färbende Zusammensetzung, die in einem kosmetisch akzeptablen Medium einen oder mehrere Farbstoffvorprodukte von Oxidationsfarbstoffen, ein Reduktionsmittel und einen aromatischen Farbstoff enthält, der durch das Reduktionsmittel zersetzt werden kann, wobei der Farbstoff in den Partikeln eines synthetischen Polymers mit Arylgruppen und einer Glasübergangstemperatur ($T_g$) von 45 °C oder darüber enthalten ist, das S-S-Bindungen und/oder Bindungen Si-C$_{aromatisch}$ aufweist, und
• eine oxidierende Zusammensetzung, die Wasserstoffperoxid enthält, und

das so hergestellte Gemisch auf die Keratinfasern aufgetragen wird, während einer Zeitspanne, die ausreichend ist, um die gewünschte Färbung zu erzeugen, einwirken gelassen wird und danach die Keratinfasern gespült, gewaschen und getrocknet werden.

20. Verfahren nach Anspruch 19, **dadurch gekennzeichnet, dass** es sich bei dem Reduktionsmittel um Natriummetabisulfit oder ein Thiol handelt.

21. Kit zur kosmetischen Behandlung von Keratinsubstanzen und insbesondere menschlichen Keratinsubstanzen, wie Haaren, Hautanhangsgebilden und der Haut, das mindestens zwei Abteilungen (A) und (B) aufweist, die die Zusammensetzungen (a) bzw. (b) enthalten, wie sie in einem der vorhergehenden Ansprüche definiert wurden.

22. Kit nach Anspruch 21, **dadurch gekennzeichnet, dass** die Zusammensetzung (a) in einem kosmetisch akzeptablen Medium ein oder mehrere Farbstoffvorprodukte von Oxidationsfarbstoffen, ein Reduktionsmittel und einen aromatischen Farbstoff enthält, der vorzugsweise wasserlöslich ist und der von dem Reduktionsmittel zersetzt werden kann, wobei der Farbstoff in den Partikeln eines synthetischen Polymers mit Arylgruppen eingeschlossen ist, das eine Glasübergangstemperatur ($T_g$) von 45 °C oder darüber aufweist und Bindungen S-S und/ oder Bindungen Si-Phenyl besitzt, und die Zusammensetzung (b) Wasserstoffperoxid enthält.

## Claims

1. Cosmetic process for treating keratin materials, in particular human keratin materials such as the hair, the integuments and the skin, comprising the simultaneous or consecutive application of two compositions (a) and (b),

• composition (a) containing particles comprising:

(i) at least one cosmetic active principle comprising one or more aromatic, carbocyclic or heterocyclic, monocyclic or fused polycyclic groups, with a molecular weight of less than or equal to 1000, and
(ii) at least one synthetic polymer containing aryl groups with a glass transition temperature ($T_g$) of greater than or equal to 45°C and comprising covalent bonds capable of being cleaved by a reagent R present in composition (b), and

• composition (b) comprising at least one reagent R capable of cleaving certain covalent bonds of the aryl-containing polymer forming the particles of composition (a),

the chemical cleavage of the bonds of the aryl-containing polymer forming the particles present in composition (a) with the reagent R present in composition (b) resulting in a release of the aromatic cosmetic active principle.

2. Process according to Claim 1, **characterized in that** the cosmetic active principle(s) (i) is (are) water-soluble.

3. Process according to Claim 1 or 2, **characterized in that** the particles are composite particles in which the cosmetic active principle(s) (i) is (are) dispersed or dissolved in the polymer matrix.

4. Process according to any one of the preceding claims, **characterized in that** composition (a) also contains a chemical agent capable of degrading the said cosmetic active principle.

5. Process according to any one of the preceding claims, **characterized in that** the synthetic polymer containing aryl groups is a nonionic polymer.

6. Process according to any one of the preceding claims, **characterized in that** the glass transition temperature of the synthetic polymer containing aryl groups is greater than 50°C and preferably greater than 60°C.

7. Process according to any one of the preceding claims, **characterized in that** the covalent bonds of the aryl-containing polymer, which are capable of being cleaved by the reagent R, are $Si-C_{aromatic}$ bonds and/or disulphide bonds (S-S).

8. Process according to Claim 7, **characterized in that** the polymer containing aryl groups is a polyorganosiloxane comprising aryl groups linked directly to the silicon atoms of the siloxane skeleton.

9. Process according to Claim 8, **characterized in that** the ratio of the number of aryl groups to the number of silicon atoms in the polymer is between 1/15 and 2/1.

10. Process according to Claim 7, **characterized in that** the synthetic polymer containing aryl groups is a polyurethane obtained by polycondensation of at least one diisocyanate and of at least one compound comprising two functions containing labile hydrogen, these monomers being chosen such that at.least one type of comonomer comprises an aryl group and at least one type of comonomer comprises a disulphide bond (S-S).

11. Process according to Claim 7, **characterized in that** the synthetic polymer containing aryl groups is a polyester or polyamide obtained by polycondensation of at least one diacid or of an activated derivative of a diacid, and, respectively, of at least one diol or of at least one diamine, these comonomers being chosen such that at least one type of comonomer comprises an aryl group and at least one type of comonomer comprises a disulphide bond (S-S).

12. Process according to any one of the preceding claims, **characterized in that** the reagent R is hydrogen peroxide ($H_2O_2$).

13. Process according to any one of the preceding claims, **characterized in that** the aromatic cosmetic active principle has a molecular weight of less than or equal to 500.

14. Process according to any one of the preceding claims, **characterized in that** the aromatic cosmetic active principle bears at least one ionized or ionizable group.

15. Process according to any one of the preceding claims, **characterized in that** the weight ratio of the cosmetic active principle to the aryl-containing polymer is between 1/1 and 1/50 and preferably between 1/3 and 1/20.

16. Process according to any one of the preceding claims, **characterized in that** the cosmetic active principle is a direct hair dye, an oxidation dye precursor, an organic UV-screening agent or a flavonoid.

17. Process according to any one of the preceding claims, **characterized in that** the particles present in composition (a) are microparticles with a mean size of between 0.05 and 500 $\mu$m or particles with a mean size of between 0.5 and 10 mm.

18. Process according to Claim 17, **characterized in that** the particles or microparticles have a core-shell structure in which the core is formed from or contains the said aromatic cosmetic active principle and the shell comprises or is formed from the said synthetic polymer containing aryl groups.

19. Process according to any one of the preceding claims, **characterized in that** the following are mixed together, immediately before use:

> • a dye composition comprising, in a cosmetically acceptable medium, one or more oxidation dye precursors, a reducing agent and an aromatic dye which can be degraded by the said reducing agent, this dye being enclosed in particles of a synthetic polymer containing aryl groups, with a glass transition temperature ($T_g$) of greater than or equal to 45°C and comprising S-S bonds and/or $Si-C_{aromatic}$ bonds, and
> • an oxidizing composition containing hydrogen peroxide,

the mixture thus obtained is applied to keratin fibres, it is left in place for a time that is sufficient to obtain the desired coloration, and the keratin fibres are rinsed, washed and dried.

20. Process according to Claim 19, **characterized in that** the reducing agent is sodium metabisulphite or a thiol.

21. Kit for the cosmetic treatment of keratin materials, in particular human keratin materials such as the hair, the integuments and the skin, comprising at least two compartments (A) and (B) containing, respectively, compositions (a) and (b) as defined in any one of the preceding claims.

22. Kit according to Claim 21, **characterized in that** composition (a) contains, in a cosmetically acceptable medium, one or more oxidation dye precursors, a reducing agent and an aromatic dye, which is preferably water-soluble and which can be degraded by the reducing agent, this dye being enclosed in particles of a synthetic polymer containing aryl groups, with a glass transition temperature ($T_g$) of greater than or equal to 45°C and comprising S-S bonds and/or Si-phenyl bonds, and composition (b) contains hydrogen peroxide.